# FASCICULE DE BREVET EUROPEEN

(11) **EP 0 623 339 B1**
(45) Date de publication et mention de la délivrance du brevet: **23.07.1997**
(21) Numéro de dépôt: 94400934.9
(22) Date de dépôt: 29.04.1994
(51) Int. Cl.: A61K 7/48

(54) **Utilisation de dérivés de la resorcine substitués en position(s)4,4 et 5 ou 4 et 6 dans des compositions cosmétiques ou dermopharmaceutiques à action dépigmentante**
Verwendung von 4-,4- und 5-, oder 4- und 6-substituierten Resorcin-Derivaten in kosmetischen oder dermopharmazeutischen Depigmentierungsmitteln
Use of 4-,4- and 5-, or 4- and 6-substituted derivatives of resorcin in cosmetic or dermopharmaceutical compositions for depigmentation

(30) Priorité: 29.04.1993 FR 9305076
(43) Date de publication de la demande: 09.11.1994
(73) Titulaire: L'OREAL, 75008 Paris (FR)
(72) Inventeur: Lagrange, Alain, F-77700 Coupvray (FR)
(74) Mandataire: Stalla-Bourdillon, Bernard

(56) Documents cités:
- EP-A- 0 341 664
- EP-A- 0 526 302

## Description

La présente invention a pour objet l'utilisation de dérivés de résorcine dans des compositions cosmétiques ou dermatologiques par application topique dans le but de blanchir la peau ou de traiter les taches pigmentaires.

On rappellera que le mécanisme de formation de la pigmentation de la peau, c'est-à-dire de la formation des mélanines est particulièrement complexe et fait intervenir schématiquement les principales étapes suivantes :
Tyrosine ---> Dopa ---> Dopaquinone ---> Dopachrome ---> Mélanines
l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat voir dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

L'utilisation de substances dépigmentantes topiques présentant une bonne efficacité et étant inoffensives, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaire à la contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo où, à défaut de pouvoir repigmenter la peau lésée, on achève de dépigmenter les zones de peau normale résiduelle pour donner à l'ensemble de la peau une teinte blanche homogène.

Après de nombreuses études sur différentes substances, on a maintenant constaté de façon tout à fait surprenante que certains dérivés de résorcine portant des substituants bien déterminés en position 4, avaient également une excellente action dépigmentante , qui pour la plupart, s'est avérée être supérieure à celle de l'hydroquinone dans le test d'inhibition "in vitro" de l'activité de la tyrosinase comme ceci sera décrit ci-après.

L'état de la technique concernant les dérivés de résorcine pour leur action dépigmentante est essentiellement constitué par la demande de brevet EP 0341 664 qui décrit des 4-alkyle résorcines. Toutefois, il s'est avéré que ceux-ci par rapport aux dérivés de résorcine selon l'invention ne présentaient pas tous une bonne action dépigmentante notamment lorsque le nombre d'atomes de carbone du radical alkyle augmentait.

La présente invention a donc pour objet l'utilisation à titre de composés actifs de dérivés de résorcine pour la préparation d'une composition cosmétique ou dermatologique ayant une action dépigmentante, les dits dérivés répondant à la formule générale suivante : 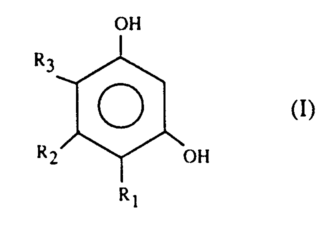 dans laquelle :
R₁ représente un atome d'halogène ou un radical -CₙH₂ₙ-CO₂R₄, n étant un nombre entier compris entre 1 et 5, R₄ représente un atome d'hydrogène ou un radical alkyle ayant de 1 à 6 atomes de carbone,
R₂ représente un atome d'hydrogène, un radical alkyle ayant de 1 à 12 atomes de carbone, hydroxyalkyle ayant de 1 à 4 atomes de carbone, formyle ou carboxy, et
R₃ représente un atome d'hydrogène, un radical alkyle ayant de 1 à 12 atomes de carbone, hydroxyalkyle ayant de 1 à 4 atomes de carbone ou carboxy, sous réserve que si R₂ est différent d'un atome d'hydrogène, R₃ représente alors un atome d'hydrogène et que si R₃ est différent d'un atome d'hydrogène, R₂ représente alors un atome d'hydrogène.

Selon l'invention, l'atome d'halogène est choisi parmi le chlore, le brome, le fluor et l'iode, mais de préférence parmi le chlore ou le brome.

Parmi les composés de formule (I), on peut citer notamment les suivants :
- la 4-bromo résorcine,
- la 4-chloro résorcine,
- la 4-iodo résorcine,
- la 4-fluoro résorcine,
- la 4-fluoro-5-formyl résorcine,
- la 4-chloro-5-carboxy résorcine,
- la 4-bromo-5-carboxy résorcine,
- la 4-bromo-5-n-pentyl résorcine,
- la 4-fluoro-5-méthyl résorcine,
- la 4-chloro-5-méthyl résorcine,
- la 4-chloro-5-hydroxyméthyl résorcine,
- la 4-chloro-6-éthyl résorcine,
- la 4-chloro-6-n-propyl résorcine,
- la 4-chloro-6-n-butyl résorcine,
- la 4-chloro-6-pentyl résorcine,
- la 4-chloro-6-isopentyl résorcine,
- la 4-chloro-6-hexyl résorcine,
- la 4-chloro-6-heptyl résorcine,
- la 4-chloro-6-octyl résorcine,
- la 4-chloro-6-décyl résorcine,
- la 4-chloro-6-dodécyl résorcine,
- la 4-chloro-6-(2-éthyl butyl) résorcine,
- la 4-chloro-6-(2-éthyl hexyl) résorcine,
- la 4-chloro-6-(1-méthyl heptyl) résorcine,
- la 4-bromo-6-éthyl résorcine,
- la 4-bromo-6-butyl résorcine,
- la 4-bromo-6-(2-éthyl hexyl) résorcine,
- la 4-bromo-6-carboxy résorcine,
- la 4-(2-carboxyéthyl) résorcine,
- la 4-(2-méthoxycarbonyl éthyl) résorcine,
- la 4-(2-isopropoxycarbonyl éthyl) résorcine,
- la 4-(3-carboxypropyl) résorcine,
- la 4-(3-méthoxycarbonyl propyl) résorcine,
- la 4-(carboxyméthyl) résorcine,
- la 4-(méthoxycarbonyl méthyl) résorcine,
- la 4-(éthoxycarbonyl méthyl) résorcine,
- la 4-(4-carboxybutyl) résorcine, et
- la 4-(4-méthoxycarbonyl butyl) résorcine.

Parmi les composés particulièrement préférés, on peut citer notamment :
- la 4-chloro résorcine,
- la 4-bromo résorcine, et
- la 4-(2-méthoxycarbonyl éthyl) résorcine.

Dans les compositions dépigmentantes selon l'invention, la concentration en composés de formule (I) est généralement comprise entre 0,01 % et 10 % et de préférence entre 0,5 % et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Ces compositions sont appliquées par voie topique en quantité correspondant aux doses d'application usuelles pour le type de composition considéré (gel, crème, lotion, etc...). Par exemple dans le cas d'une crème on utilise de 0,5 à 3 mg et notamment de 1 à 2 mg de crème par cm2 de peau et par application, à raison d'une ou deux applications par jour.

Les composés de formule générale (I) sont pour la plupart connus et sont obtenus selon les méthodes de synthèse conventionnelles.

### Etudes "in vitro"

Certains des composés de formule générale (I) ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

### Protocole expérimental

### Réactifs :

A - Tampon phosphate 0,1 M pH = 6,5 (Tween 20 à 1 %)
B - Solution mère de L-tyrosine à 2.10⁻³ M dans A
C - Solution mère de L-dopa à 10⁻⁴ M dans A
D - Solution mère de tyrosinase de champignons à 2.400 unités/ml dans A
E - Solution mère de l'inhibiteur à 10⁻² M dans A (les solutions C et D sont à préparer le jour même).

### Résultats :

- cuve de référence : 3 ml de A
- cuve d'essai : 1 ml de B
   0,1 ml de C
   1,85ml de A + E
- homogénéiser et équilibrer à 25°C
- ajouter 0,05 ml de D
- mélanger rapidement et observer la cinétique par la mesure de l'absorbance à 475 nm en fonction du temps.

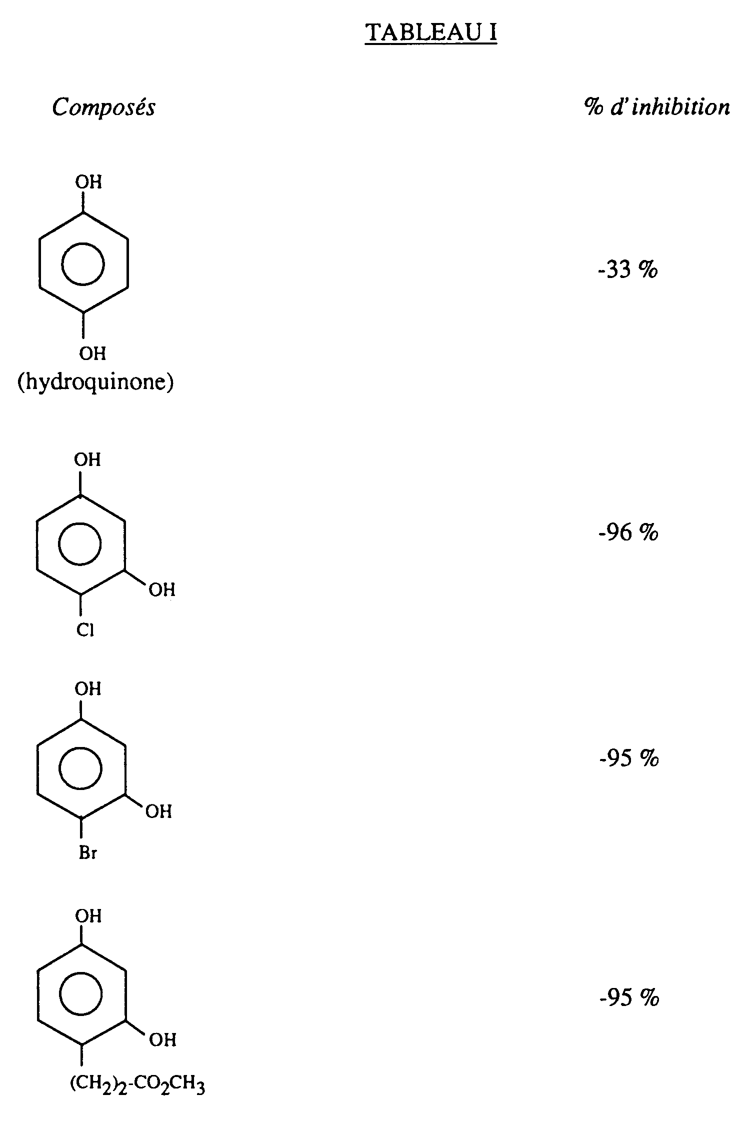

### EXEMPLES DE COMPOSITION

### EXEMPLE 1 : Crème dépigmentante

| | |
|---|---|
| - 4-chloro résorcine | 2,5 g |
| - Alcool cétylstéarylique oxyéthyléné à 12 moles d'oxyde d'éthylène | 0,5 g |
| - Alcool cétylstéarylique oxyéthyléné à 20 moles d'oxyde d'éthylène | 0,5 g |
| - Monostéarate de glycol | 3 g |
| - Mélange de caprylate-caprate de coprah | 5,0 g |
| - Polymère d'acide acrylique réticulé vendu sous la dénomination de "Carbomer 934P" par la Société Goodrich | 0,3 g |
| - Triéthanolamine | 0,9 g |
| - Ethanol | 25 g |
| - Glycérine | 3 g |
| - Parfum, conservateurs q.s. | |
| - Eau q.s.p. | 100 g |

Dans cet exemple, la 4-chloro résorcine peut être remplacée par 3,0 g de 4-(2-méthoxycarbonyl éthyl) résorcine.

### EXEMPLE 2 : Lotion dépigmentante

| | |
|---|---|
| - 4-chloro résorcine | 2,5 g |
| - Ethanol | 50 g |
| - Polyéthylèneglycol 400 | 30 g |
| - Ethoxy diglycol | 5 g |
| - Glycérine | 5 g |
| - Eau q.s.p. | 100 g |

Dans cet exemple, la 4-chloro résorcine peut être remplacée par 2,0 g de 4-bromo résorcine.

## Revendications

1. Utilisation à titre de composés actifs de dérivés de résorcine pour la préparation d'une composition cosmétique ou dermatologique ayant une action dépigmentante, caractérisée par le fait que les dits dérivés répondent à la formule suivante : 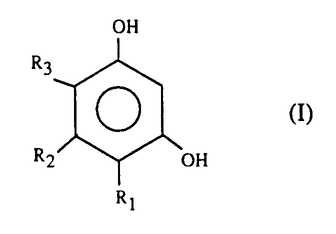 dans laquelle :
R₁ représente un atome d'halogène ou un radical -CₙH₂ₙ-CO₂R₄, n étant un nombre entier compris entre 1 et 5, et R₄ représente un atome d'hydrogène ou un radical alkyle ayant de 1 à 6 atomes de carbone,
R₂ représente un atome d'hydrogène, un radical alkyle ayant de 1 à 12 atomes de carbone, hydroxyalkyle ayant de 1 à 4 atomes de carbone, formyle ou carboxy, et
R₃ représente un atome d'hydrogène, un radical alkyle ayant de 1 à 12 atomes de carbone, hydroxyalkyle ayant de 1 à 4 atomes de carbone ou carboxy, sous réserve que si R₂ est différent d'un atome d'hydrogène, R₃ représente alors un atome d'hydrogène et que si R₃ est différent d'un atome d'hydrogène, R₂ représente alors un atome d'hydrogène.

2. Utilisation selon la revendication 1, caractérisée par le fait que lesdits dérivés de formule (I) sont choisis dans le groupe constitué par :
- la 4-bromo résorcine,
- la 4-chloro résorcine,
- la 4-iodo résorcine,
- la 4-fluoro résorcine,
- la 4-fluoro-5-formyl résorcine,
- la 4-chloro-5-carboxy résorcine,
- la 4-bromo-5-carboxy résorcine,
- la 4-bromo-5-n-pentyl résorcine,
- la 4-fluoro-5-méthyl résorcine,
- la 4-chloro-5-méthyl résorcine,
- la 4-chloro-5-hydroxyméthyl résorcine,
- la 4-chloro-6-éthyl résorcine,
- la 4-chloro-6-n-propyl résorcine,
- la 4-chloro-6-n-butyl résorcine,
- la 4-chloro-6-pentyl résorcine,
- la 4-chloro-6-isopentyl résorcine,
- la 4-chloro-6-hexyl résorcine,
- la 4-chloro-6-heptyl résorcine,
- la 4-chloro-6-octyl résorcine,
- la 4-chloro-6-décyl résorcine,
- la 4-chloro-6-dodécyl résorcine,
- la 4-chloro-6-(2-éthyl butyl) résorcine,
- la 4-chloro-6-(2-éthyl hexyl) résorcine,
- la 4-chloro-6-(1-méthyl heptyl) résorcine,
- la 4-bromo-6-éthyl résorcine,
- la 4-bromo-6-butyl résorcine,
- la 4-bromo-6-(2-éthyl hexyl) résorcine,
- la 4-bromo-6-carboxy résorcine,
- la 4-(2-carboxyéthyl) résorcine,
- la 4-(2-méthoxycarbonyl éthyl) résorcine,
- la 4-(2-isopropoxycarbonyl éthyl) résorcine,
- la 4-(3-carboxypropyl) résorcine,
- la 4-(3-méthoxycarbonyl propyl) résorcine,
- la 4-(carboxyméthyl) résorcine,
- la 4-(méthoxycarbonyl méthyl) résorcine,
- la 4-(éthoxycarbonyl méthyl) résorcine,
- la 4-(4-carboxybutyl) résorcine, et
- la 4-(4-méthoxycarbonyl butyl) résorcine.

3. Utilisation selon l'une quelconque des revendications précédentes, caractérisée par le fait que lesdits dérivés de formule (I) sont choisis de préférence parmi la 4-chloro résorcine, la 4-bromo résorcine et la 4-(2-méthoxycarbonyl éthyl) résorcine.

4. Utilisation selon l'une quelconque des revendications précédentes, caractérisée par le fait que la concentration en dérivés de formule (I) dans la composition est comprise entre 0,01 % et 10 % en poids par rapport au poids total de la composition.

5. Utilisation selon l'une quelconque des revendications précédentes, caractérisée par le fait que la concentration en dérivés de formule (I) dans la composition est de préférence comprise entre 0,5 % et 5 % en poids par rapport au poids total de la composition.

6. Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

7. Utilisation selon l'une quelconque des revendications précédentes, caractérisées par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

## Claims

1. Use as active compounds of resorcinol derivatives for preparing a cosmetic or dermatological composition having a depigmenting action, characterized in that the said derivatives correspond to the following formula: 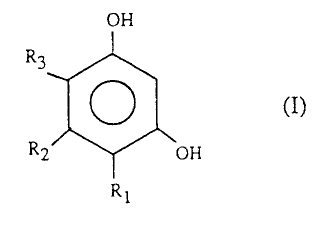 in which:
R₁ represents a halogen atom or a radical -CₙH₂ₙ-CO₂R₄, n being an integer between 1 and 5 and R₄ representing a hydrogen atom or an alkyl radical having from 1 to 6 carbon atoms,
R₂ represents a hydrogen atom, an alkyl radical having from 1 to 12 carbon atoms, a hydroxyalkyl radical having from 1 to 4 carbon atoms, a formyl or carboxyl radical, and
R₃ represents a hydrogen atom, an alkyl radical having from 1 to 12 carbon atoms, a hydroxyalkyl radical having from 1 to 4 carbon atoms or a carboxyl radical, with the proviso that, if R₂ is other than a hydrogen atom, R₃ then represents a hydrogen atom and, if R₃ is other than a hydrogen atom, R₂ then represents a hydrogen atom.

2. Use according to Claim 1, characterized in that the said derivatives of formula (I) are chosen from the group consisting of:
- 4-bromoresorcinol,
- 4-chlororesorcinol,
- 4-iodoresorcinol,
- 4-fluororesorcinol,
- 4-fluoro-5-formylresorcinol,
- 4-chloro-5-carboxyresorcinol,
- 4-bromo-5-carboxyresorcinol,
- 4-bromo-5-n-pentylresorcinol,
- 4-fluoro-5-methylresorcinol,
- 4-chloro-5-methylresorcinol,
- 4-chloro-5-hydroxymethylresorcinol,
- 4-chloro-6-ethylresorcinol,
- 4-chloro-6-n-propylresorcinol,
- 4-chloro-6-n-butylresorcinol,
- 4-chloro-6-pentylresorcinol,
- 4-chloro-6-isopentylresorcinol,
- 4-chloro-6-hexylresorcinol,
- 4-chloro-6-heptylresorcinol,
- 4-chloro-6-octylresorcinol,
- 4-chloro-6-decylresorcinol,
- 4-chloro-6-dodecylresorcinol,
- 4-chloro-6-(2-ethylbutyl)resorcinol,
- 4-chloro-6-(2-ethylhexyl)resorcinol,
- 4-chloro-6-(1-methylheptyl)resorcinol,
- 4-bromo-6-ethylresorcinol,
- 4-bromo-6-butylresorcinol,
- 4-bromo-6-(2-ethylhexyl)resorcinol,
- 4-bromo-6-carboxyresorcinol,
- 4-(2-carboxyethyl)resorcinol,
- 4-(2-methoxycarbonylethyl)resorcinol,
- 4-(2-isopropoxycarbonylethyl)resorcinol,
- 4-(3-carboxypropyl)resorcinol,
- 4-(3-methoxycarbonylpropyl)resorcinol,
- 4-(carboxymethyl)resorcinol,
- 4-(methoxycarbonylmethyl)resorcinol,
- 4-(ethoxycarbonylmethyl)resorcinol,
- 4-(4-carboxybutyl)resorcinol, and
- 4-(4-methoxycarbonylbutyl)resorcinol.

3. Use according to either of the preceding claims, characterized in that the said derivatives of formula (I) are preferably chosen from 4-chlororesorcinol, 4-bromoresorcinol and 4-(2-methoxycarbonylethyl)resorcinol.

4. Use according to any one of the preceding claims, characterized in that the concentration of derivatives of formula (I) in the composition is between 0.01 % and 10 % by weight relative to the total weight of the composition.

5. Use according to any one of the preceding claims, characterized in that the concentration of derivatives of formula (I) in the composition is preferably between 0.5 % and 5 % by weight relative to the total weight of the composition.

6. Use according to any one of the preceding claims, characterized in that the cosmetic or dermatological composition takes the form of a lotion, a cream, a milk, a gel, a mask, microspheres or nanospheres or vesicle dispersions.

7. Use according to any one of the preceding claims, characterized in that the cosmetic or dermatological composition additionally contains a moisturizing agent, a surface-active agent, a keratolytic agent, an anti-inflammatory agent, a complexing agent, an antioxidant, a preservative, a perfume or a sunscreen.

## Patentansprüche

1. Verwendung von Resorcinderivaten als Wirkstoffe zur Herstellung einer kosmetischen oder dermatologischen Zubereitung mit einer depigmentierenden Wirkung, dadurch gekennzeichnet, daß diese Derivate der folgenden Formel entsprechen: 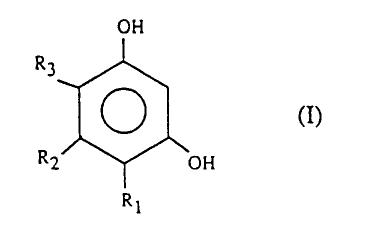 worin
R₁ ein Wasserstoffatom oder einen Rest -CₙH₂ₙ-CO₂R darstellt, wobei n eine ganze Zahl zwischen 1 und 5 und R₄ ein Wasserstoffatom oder einen Alkylrest mit 1 bis 6 Kohlenstoffatomen bedeuten,
R₂ ein Wasserstoffatom, einen Alkylrest mit 1 bis 12 Kohlenstoffatomen, einen Hydroxyalkylrest mit 1 bis 4 Kohlenstoffatomen, eine Formyl- oder Carboxygruppe darstellt, und
R₃ ein Wasserstoffatom, einen Alkylrest mit 1 bis 12 Kohlenstoffatomen, einen Hydroxyalkylrest mit 1 bis 4 Kohlenstoffatomen, oder die Carboxygruppe bedeutet, und zwar mit der Maßgabe, daß dann, wenn R₂ von einem Wasserstoffatom verschieden ist, R₃ ein Wasserstoffatom darstellt, und in dem Falle, daß R₃ von einem Wasserstoffatom verschieden ist, R₂ dann ein Wasserstoffatom bedeutet.

2. Verwendung nach Anspruch 1, dadurch gekennzeichnet, daß die Derivate gemäß der Formel (I) aus der aus den folgenden Verbindungen bestehenden Gruppe ausgewählt werden:
4-Bromresorcin,
4-Chlorresorcin,
4-Jodresorcin,
4- Fluorresorcin,
4-Fluor-5-formylresorcin,
4-Chlor-5-carboxyresorcin,
4-Brom-5-carboxyresorcin,
4-Brom-5-n-pentylresorcin,
4-Fluor-5-methylresorcin,
4-Chlor-5-methylresorcin,
4-Chlor-5-hydroxymethylresorcin,
4-Chlor-6-ethylresorcin,
4-Chlor-6-n-propylresorcin,
4-Chlor-6-n-butylresorcin,
4-Chlor-6-pentylresorcin,
4-Chlor-6-isopentylresorcin,
4-Chlor-6-hexylresorcin,
4-Chlor-6-heptylresorcin,
4-Chlor-6-octylresorcin,
4-Chlor-6-decylresorcin,
4-Chlor-6-dodecylresorcin,
4-Chlor-6-(2-ethylbutyl)-resorcin,
4-Chlor-6-(2-ethylhexyl)-resorcin,
4-Chlor-6-(1-methylheptyl)-resorcin,
4-Brom-6-ethylresorcin,
4-Brom-6-butylresorcin,
4-Brom-6-(2-ethylhexyl)-resorcin,
4-Brom-6-carboxyresorcin,
4-(2-Carboxyethyl)-resorcin,
4-(2-Methoxycarbonylethyl)-resorcin,
4-(2-Isopropoxycarbonylethyl)-resorcin,
4-(3-Carboxypropyl)-resorcin,
4-(3-Methoxycarbonylpropyl)-resorcin,
4-(Carboxymethyl)-resorcin,
4-(Methoxycarbonylmethyl)-resorcin,
4-(Ethoxycarbonylmethyl)-resorcin,
4-(4-Carboxybutyl)-resorcin, sowie
4-(4-Methoxycarbonylbutyl)-resorcin.

3. Verwendung nach einem der vorstehenden Ansprüche, dadurch gekennzeichnet, daß die Derivate gemäß der Formel (I) vorzugsweise unter 4-Chlorresorcin, 4-Bromresorcin und 4-(2-Methoxycarbonylethyl)-resorcin ausgewählt werden.

4. Verwendung nach einem der vorstehenden Ansprüche, dadurch gekennzeichnet, daß die Konzentration der Derivate gemäß der Formel (I) in der Zubereitung zwischen 0,01 Gew.-% und 10 Gew.-% in bezug auf das Gesamtgewicht der Zubereitung beträgt.

5. Verwendung nach einem der vorstehenden Ansprüche, dadurch gekennzeichnet, daß die Konzentration an Derivaten gemäß der Formel (I) in der Zubereitung vorzugsweise zwischen 0,5 Gew.-% und 5 Gew.-% in bezug auf das Gesamtgewicht der Zubereitung beträgt.

6. Verwendung nach einem der vorstehenden Ansprüche, dadurch gekennzeichnet, daß die kosmetische oder dermatologische Zubereitung in Form einer Lotio, Creme, Milch, eines Gels, einer Maske, in Form von Mikrokügelchen oder Nanokügelchen oder auch in Form von vesikulären Dispersionen vorliegt.

7. Verwendung nach einem der vorstehenden Ansprüche, dadurch gekennzeichnet, daß die kosmetische oder dermatologische Zubereitung zusätzlich noch ein Feuchthaltemittel, ein Tensid, ein Keratolytikum, einen Entzündungshemmer, ein Komplexierungsmittel, ein Antioxidans, ein Konservierungsmittel, einen Duftstoff oder eine Sonnenfiltersubstanz enthält.
